Europäisches Patentamt

(19) European Patent Office

Office européen des brevets

(11) Publication number: **0 085 469**
**B1**

## (12) EUROPEAN PATENT SPECIFICATION

(45) Date of publication of patent specification: **30.07.86**

(21) Application number: **83200174.7**

(22) Date of filing: **01.02.83**

(51) Int. Cl.⁴: **A 61 K 39/102** // A61K39/10

(54) Method of preparing an immunogen of Pasteurella multocida.

(30) Priority: **03.02.82 NL 8200392**

(43) Date of publication of application:
**10.08.83 Bulletin 83/32**

(45) Publication of the grant of the patent:
**30.07.86 Bulletin 86/31**

(84) Designated Contracting States:
**AT BE CH DE FR GB IT LI LU NL SE**

(56) References cited:
**GB-A-1 441 098**

**BIOLOGICAL ABSTRACTS, vol. 63, ref. 63249,
1977, Philadelphia, PA. (USA); K.K.
SRIVASTAVA et al.: "Characterization of an
immunogenic fraction of Pasteurella multocida
culture filtrates".**

**J. COMP. PATH., vol. 76, 1966, pages 303-314;
G.H. PRINCE et al.: "Antigenic studies on
pasteurella multocida using immunodiffusion
techniques. I. Identification and nomenclature
of the soluble antigens of a bovine
haemorrhagic septicaemia strain".**

(73) Proprietor: **DUPHAR INTERNATIONAL
RESEARCH B.V
C.J. van Houtenlaan 36
NL-1381 CP Weesp (NL)**

(72) Inventor: **Pranger, Maarten Hendrik
OCTROOIBUREAU ZOAN B.V. Apollolaan 151
NL-1077 AR Amsterdam (NL)**
Inventor: **De Wachter, Jan Cornelis
OCTROOIBUREAU ZOAN B.V. Apollolaan 151
NL-1077 AR Amsterdam (NL)**

(74) Representative: **Muis, Maarten et al
OCTROOIBUREAU ZOAN B.V. P.O. Box 140
NL-1380 AC Weesp (NL)**

(56) References cited:
**ZBL. VET. MED., vol. 27, 1980, pages 125-130,
Verlag Paul Parey, Berlin (DE); W. SCHULLER
et al.: "Vaccination against atrophic rhinitis in
swine with a combined bordetella
bronchiseptica, pasteurella multocida vaccine".**

# Description

The invention relates to a method of preparing an immunogen by cultivating a *Pasteurella multocida*-strain and gaining the culture-filtrate.

Such a method is known from the article by K. K. Srivastava et al, Can. J. Microbiology, Vol. *23*, (1977), 197—201. In this article it is described that a culture-filtrate and a certain fraction thereof, respectively, provides some protection in mice when these are infected, after immunization, with 25 $LD_{50}$ *Pasteurella multocida* bacteria.

Further J. Comp. Path. 1966, Vol. *76*, pages 303—314 relates to a study for establishing standard immunodiffusion systems for the purpose of enabling identification and nomenclature of antigens. Moreover, some of the properties of the antigens are mentioned.

Finally several bacterin vaccines have been described and tested, among others in Zbl. Vet. Med. B. *27*, (1980) pages 125—130). The described experimental vaccine is a combination of *Bordetella bronchiseptica* and *Pasteurella multocida* bacterin, consisting of glutaraldehyde-inactivated whole organisms, and aluminium hydroxide as an adjuvant. This vaccine gives a reasonable protection of piglets against atrophic rhinitis through the colostrum of vaccinated sows. However, no results obtained after challenging the piglets are mentioned.

It has now been found that a very active immunogen can be obtained by cultivating a *Pasteurella multocida* strain for 12—24 hours, preferably for 14—16 hours, in a liquid medium, removing the bacteria from the culture medium, sterile filtering the culture filtrate and rapidly freezing or freeze-drying it, so as to avoid inactivation.

It is essential for the strain to be cultivated for at most 24 hours, since in the case of longer-lasting cultivation, the immunogenicity does not increase or scarcely increases, or even decreases, while the toxicity of the culture filtrate in the case of longer cultivation increases rapidly in particular when toxin-producing strains are used.

The cultivation of the strain can be carried out in a manner known per se in a liquid cultivation medium suitable for this purpose at 37°C. A suitable cultivation medium is, for example, brain-heart-infusion medium (BHI-medium).

It has been found that the immunogen in the culture filtrate to be obtained according to the above method is very sensitive to both chemical and thermal inactivation. When the liquid culture filtrate is kept at 60°C for 30 minutes, or at +4°C for a longer period of time, or kept frozen at −20°C, the immunogenicity of the composition thus treated decreases considerably. The immunogenic activity also decreases by a treatment with 0.3% formalin for 24 hours or with thimerosal (1:10,000).

Various *Pasteurella multocida* strains may be used as starting materials. For example, good results are obtained by cultivating in the above-described manner, and further treating as starting materials, strains isolated from pigs (for example, the strain of the Carter D-type which is known under No. 47459-449 at the Central Diergeneeskundig Instituut (CDI) (Central Veterivary Institute), or the strain of the Carter type A which is known under No. 53566 at the CDI), or a strain isolated from turkeys (for example, the strain 3 USA, standard type 3, P-1059).

The following properties of the immunogen to be obtained according to the invention may be mentioned:

a) in the wet state the immunogen is very thermolabile,

b) in the freeze-dried state the immunogen is comparatively insensitive to temperature (i.e. stable);

c) the immunogen is sensitive to chemical inactivation with, formalin or thimerosal;

d) the immunogen to be obtained according to the invention may be used in known manner in vaccins for the control of infections caused by *Pasteurella multocida*, for example, atrophic rhinitis in pigs, fowl cholera in poulty, haemorrhagic septicaemia in cattle and pasteurellosis c.q. pasteurella infections in sheep, cattle, pigs, rabbits and man;

e) the immunogen may be used for the preparation of combination vaccines, for example, in a vaccin against atrophic rhinitis it may be combined with *Bordetella bronchiseptica* antigen;

f) by the addition of adjuvants the protective action can be even further increased.

The invention will now be described in greater detail with reference to the following examples in which the immunogenic activity of the culture filtrate obtained is demonstrated both in direct and in indirect protection tests.

Example I

A freeze-dried inoculation culture of a *Pasteurella multocida* strain of the Carter type D (known at the CDI under No. 47459-449) isolated from pigs was used as a starting material for the preparation of the immunogen. The contents of an inoculation ampoule, after reconstitution in distilled water, were spread on sheep blood-agar medium and incubated at 37°C.

After 16 hours the separate colonies were transferred to a starting culture of BHI-medium. After 8 hours' incubation at 37°C on a shaking machine (80 rpm), inoculation was again carried out (5% vol/vol) on BHI medium for the production culture. Incubation was carried out at 37°C for 16 hours on a shaking machine (80 rpm). The cultivated Pasteurella germs were then centrifuged at 10,000 rpm (17,000×g) for 30 minutes. The supernatant was decanted and filtered sterile; the culture filtrate thus obtained was frozen at −70°C.

Example II

Dilutions indicated in Table A were prepared from the culture filtrate (CF) obtained according to Example I.

Per dilution, 10 mice were vaccinated once intraperitoneally (i.p.) with 0.5 ml.

Two weeks after vaccination the animals were challenged i.p. with $10^4 LD_{50}$ germs of the homologous strain. The percentage of surviving animals 1 week after challenge is recorded in Table A.

TABLE A

| CF-dilution | % Survivors |
|---|---|
| $1:10^1$ | 90 |
| $1:10^2$ | 100 |
| $1:10^3$ | 90 |
| $1:10^4$ | 100 |
| $1:10^5$ | 30 |
| $1:10^6$ | 40 |
| $1:10^7$ | 0 |
| Control group | 0 |

Summarizing it may be concluded that mice, after one vaccination with 10,000× diluted culture filtrate of the strain mentioned in Example I, are protected for 100% against a challenge infection with $10^4 LD_{50}$ germs of the homologous strain.

Example III

In the manner described in Example I, a culture filtrate was prepared starting from the pig's strain of the Carter type A (known at CDI under No. 53566).

The dilutions recorded in Table B were made from this culture filtrate. Per dilution, 10 mice were vaccinated i.p. with 0.5 ml. This was repeated after a fortnight. Two weeks after the last vaccination the animals were challenged i.p. with the quantity of germs of the homologous *Pasteurella multocida* A strain mentioned in Table B. One week after challenge the percentage of animals recorded in Table B was still alive.

TABLE B

| CF-dilution | Challenge dose . . . $LD_{50}$ | % Survivors |
|---|---|---|
| 1:2 | 50 | 100 |
| 1:4 | 50 | 100 |
| 1:8 | 500 | 100 |
| 1:16 | 500 | 100 |
| 1:32 | 500 | 80 |
| 1:64 | 5,000 | 100 |
| 1:128 | 5,000 | 100 |
| 1:256 | 50,000 | 100 |
| 1:512 | 50,000 | 0 |
| Control group | 50 | 0 |

Summarizing it may be concluded that mice after two vaccinations with 256× diluted culture filtrate of the said strain are protected for 100% against a challenge infection with $5×10^4 LD_{50}$ germs of the homologous strain.

Example IV

In the manner described in Example I, a culture filtrate (CF) was prepared from material of the *Pasteurella multocida* strain 3 USA (a turkey strain known under No. P-1059).

The dilutions mentioned in Table C were made from this CF. With each dilution, 3 mice were vaccinated twice (day 0 and day 14) i.p. with 0.5 ml.

Two weeks after the last vaccination the animals were challenged i.p. with the quantity of bacteria of the homologous *Pasteurella multocida* strain 3 USA recorded in Table C.

One week after challenge the percentage of animals mentioned in Table C were still alive.

### TABLE C

| CF-dilution | Infection dose ... $LD_{50}$ | % Survival |
|---|---|---|
| 1:2 | 50 | 67 |
| 1:4 | 50 | 100 |
| 1:8 | 500 | 100 |
| 1:16 | 500 | 100 |
| 1:32 | 5,000 | 100 |
| 1:64 | 5,000 | 100 |
| 1:128 | 50,000 | 100 |
| 1:256 | 50,000 | 100 |
| 1:512 | 500,000 | 0 |
| 1:1024 | 500,000 | 33 |
| Control group | 50 | 0 |

Summarizing it may be concluded that mice, after two vaccinations with 256× diluted culture filtrate of the said strain, are protected for 100% against a challenge infection with $5×10^4$ $LD_{50}$ germs of the homologous strain.

**Claims**

1. A method of preparing an immunogen by cultivating a *Pasteurella multocida* strain, and gaining the culture filtrate, characterized in that a *Pasteurella multocida* strain is cultivated for at most 12—24 hours in a liquid medium, the bacteria are removed from the culture liquid, the culture filtrate is filtered sterile and is rapidly frozen or freeze-dried so as to avoid inactivation.

2. A method as claimed in Claim 1, characterized in that, after cultivating for 14—16 hours, the bacteria are separated by centrifuging or ultrafiltration.

3. A method of preparing a vaccine against *Pasteurella multocida* infections, characterized in that an immunogen obtained according to Claim 1 or 2 is brought into a form suitable for administration.

4. A method as claimed in Claim 3, characterized in that a vaccine is prepared against atrophic rhinitis.

5. A method as claimed in Claim 4, characterized in that *Bordetella bronchiseptica* antigen is incorporated in the vaccine.

6. A vaccine obtained according to the method as claimed in Claims 3—5.

**Revendications**

1. Un procédé pour la préparation d'un immunogène en cultivant une souche d'une *Pasteurella multocida* et obtenant le filtrat de la culture, caractérisé en ce que une souche *Pasteurella multocida* de est cultivée pendant au maximum 12 à 24 heures dans un milieu de culture liquide, les bactéries sont isolées du liquide de la culture, le filtrat de la culture est filtré d'une façon stérile et est surgelé rapidement ou lyophilisé pour éviter l'inactivation.

2. Un procédé tel que revendiqué dans la revendication 1, caractérisé en ce que les bactéries, après les avoir cultivées pendant 14 à 16 heures, sont séparées au moyen de la centrifugation ou l'ultrafiltration.

3. Un procédé pour la préparation d'un vaccin contre des infections provoquées par *Pasteurella multocida*, caractérisé en ce qu'un immunogène obtenu selon la revendication 1 ou 2, est mis en une forme apte à l'administration.

4. Un procédé tel que revendiqué dans la revendication 3, caractérisé en ce qu'un vaccin est préparé contre la rhinite atrophique.

5. Un procédé selon la revendication 4, caractérisé en ce que l'antigène *Bordetella bronchiseptica* est incorporé dans le vaccin.

6. Un vaccin obtenu selon le procédé revendiqué dans les revendications 3 à 5.

**Patentansprüche**

1. Verfahren zur Herstellung eines Immunogens durch Züchtung eines *Pasteurella multocida* Stammes, und Gewinnung des Züchtungsfiltrates, dadurch gekennzeichnet, dass ein *Pasteurella Multocida* Stamm während nicht mehr als 12 bis 24 Stunden in einem flüssigen Medium gezüchtet wird, die Bakterien aus der Züchtungsflüssigkeit enfernt werden, das Züchtungsfiltrat steril filtriert und zur Vermeidung von Inaktivierung schnell gefriert oder gefriertrochnet wird.

2. Verfahren nach Anspruch 1, dadurch gekennzeichnet, dass die Bakterien nach 14 bis 16 Stunden züchten durch zentrifugieren oder ultrazentrifugieren abgetrennt werden.

3. Verfahren zur Herstellung eines Impfstoffes gegen *Pasteurella multocida* Infektionen, dadurch gekennzeichnet, dass Immunogen hergestellt nach Anspruch 1 oder 2 in eine zur Verabrechung geeignete Form gebracht wird.

4. Verfahren nach Anspruch 3, dadurch gekennzeichnet, dass ein Impfstoff gegen atrophische Rhinitis hergestellt wird.

5. Verfahren nach Anspruch 4, dadurch gekennzeichnet, dass das *Bordetella bronchiseptica* im Impfstoff aufgenommen wird.

6. Impfstoff hergestellt nach den Verfahren beansprucht in Ansprüchen 3 bis 5.